(19) Europäisches Patentamt
European Patent Office
Office européen des brevets

(11) EP 1 257 275 B1

(12) # FASCICULE DE BREVET EUROPEEN

(45) Date de publication et mention de la délivrance du brevet:
**06.04.2005 Bulletin 2005/14**

(21) Numéro de dépôt: **01907722.1**

(22) Date de dépôt: **07.02.2001**

(51) Int Cl.[7]: **A61K 31/454**, A61P 25/34, A61K 45/06

(86) Numéro de dépôt international:
**PCT/FR2001/000356**

(87) Numéro de publication internationale:
**WO 2001/058450 (16.08.2001 Gazette 2001/33)**

(54) **UTILISATION D'UN ANTAGONISTE DES RECEPTEURS AUX CANNABINOIDES CENTRAUX POUR LA PREPARATION DE MEDICAMENTS UTILES POUR FACILITER L'ARRET DE LA CONSOMMATION DE TABAC**

VERWENDUNG EINES ZENTRALEN CANNABINOID-REZEPTOR-ANTAGONISTEN FÜR DIE HERSTELLING VON ARZNEIMITTELN ZUR ERLEICHTERUNG DER AUFGABE DES TABAKVERBRAUCHS

USE OF CENTRAL CANNABINOID RECEPTOR ANTAGONIST FOR PREPARING MEDICINES DESIGNED TO FACILITATE SMOKING CESSATION

(84) Etats contractants désignés:
**AT BE CH CY DE DK ES FI FR GB GR IE IT LI LU MC NL PT SE TR**
Etats d'extension désignés:
**AL LT LV MK RO SI**

(30) Priorité: **09.02.2000 FR 0001682**

(43) Date de publication de la demande:
**20.11.2002 Bulletin 2002/47**

(73) Titulaire: **Sanofi-Aventis**
**75013 Paris (FR)**

(72) Inventeurs:
- **BLANCHARD, Jean, Charles**
  **F-94160 Saint Mande (FR)**
- **MENARD, François**
  **F-75006 Paris (FR)**

(74) Mandataire: **Thouret-Lemaitre, Elisabeth et al**
**Sanofi-Aventis**
**174 avenue de France**
**75013 Paris (FR)**

(56) Documents cités:
**EP-A- 0 656 354**
**WO-A-00/46209**
**WO-A-98/32441**

- **KOBAYASHI H ET AL: "Recent progress in the neurotoxicology of natural drugs associated with dependence or addiction, their endogenous agonists and receptors." JOURNAL OF TOXICOLOGICAL SCIENCES, (1999 FEB) 24 (1) 1-16. REF: 96, XP000952313**
- **F. RODRIGUEZ DE FONSECA ET AL.: "Cannabinoid receptor antagonist SR141716A decreases operant ethanol self administration in rats exposed to ethanol-vapor chambers" ZHONGGUO YAOLI XUEBAO, vol. 20, no. 12, 1999, pages 1109-1114, XP000952383**
- **GJ MOLDERINGS ET AL.: "Presynaptic imidazoline receptors mediate inhibition of noradrenaline release from sympathetic nerves in rat blood vessels" FUNDAMENTAL AND CLINICAL PHARMACOLOGY, vol. 12, no. 4, 1998, pages 388-397, XP001041056**
- **LLOYD G.K. J. PHARMACOL. EXP. THERAP. vol. 292, 2000, pages 461 - 467**
- **FATTORE L. ET AL BEHAV. BRAIN. RES. vol. 104, 1999, pages 141 - 146**
- **TANDA ET AL NATURE NEUROSCIENCE vol. 3, 2000, pages 1073 - 1074**
- **COSSU G. ET AL BEHAV. BRAIN. RES. vol. 118, 2001, pages 61 - 65**
- **DATABASE OVID MEDLINE 2001 'Nicotinic agonists-Monoamine Oxidase Inhibitors'**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

EP 1 257 275 B1

Printed by Jouve, 75001 PARIS (FR)

## Description

La présente invention concerne une nouvelle utilisation d'un antagoniste des récepteurs aux cannabinoïdes centraux dits récepteurs CB1. Plus particulièrement, l'invention se rapporte à l'utilisation d'un antagoniste des récepteurs CB1 pour la préparation de médicaments utiles pour faciliter l'arrêt de la consommation de tabac.

Des familles de composés ayant une affinité pour les récepteurs aux cannabinoïdes ont été décrites dans plusieurs brevets et demandes de brevets, en particulier la demande européenne EP-576 357, qui décrit des dérivés du pyrazole, et la demande WO 96/02248 qui décrit notamment des dérivés du benzofurane.

Plus particulièrement, le N-pipéridino-5-(4-chlorophényl)-1-(2,4-dichlorophényl)-4-méthylpyrazole-3-carboxamide, ci-après dénommé composé A, de formule :

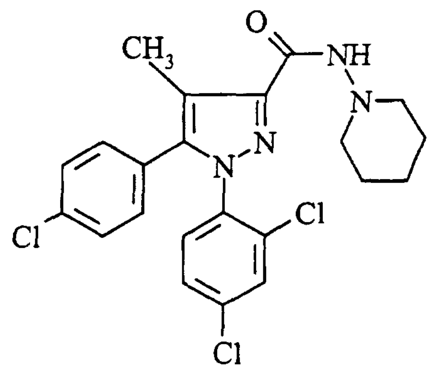

(I)

ses sels pharmaceutiquement acceptables et leurs solvats, sont décrits dans le brevet européen EP-656 354 et par M. Rinaldi-Carmona et al. (FEBS Lett., 1994, 350, 240-244), comme antagonistes des récepteurs centraux CB,.

Il est décrit que le composé A et ses sels qui sont des antagonistes des récepteurs centraux aux cannabinoïdes peuvent être utilisés pour le traitement des troubles de l'appétit, notamment en tant qu'anorexigène, et dans le traitement des troubles liés à l'utilisation de substances psychotropes. De plus, la demande internationale WO99/00119 divulgue l'utilisation des antagonistes des récepteurs aux cannabinoïdes centraux pour traiter les troubles de l'appétence, c'est-à-dire réguler les désirs de consommation, en particulier pour la consommation de sucres, de carbohydrates, d'alcool ou de drogues et plus généralement d'ingrédients appétissants.

On a maintenant trouvé que le composé A, ses sels pharmaceutiquement acceptables et leurs solvats permettent de faciliter l'arrêt de la consommation de tabac, qu'ils sont utiles dans le traitement de la dépendance à la nicotine et/ou dans le traitement des symptômes du sevrage à la nicotine.

Ainsi, l'administration du composé A, d'un de ses sels pharmaceutiquement acceptables ou solvats, permet d'observer chez des consommateurs de nicotine tels que les fumeurs de tabac, une abstinence tabagique totale ou partielle de façon précoce ou retardée. De plus, les symptômes du sevrage à la nicotine sont très sensiblement atténués voire supprimés, et la prise de poids consécutive à l'arrêt de la consommation tabagique est réduite ou inexistante.

Selon un des ses aspects, la présente invention concerne l'utilisation du N-pipéridino-5-(4-chlorophényl)-1-(2,4-dichlorophényl)-4-méthylpyrazole-3-carboxamide, d'un de ses sels pharmaceutiquement acceptables ou d'un de leurs solvats pour la préparation de médicaments utiles pour faciliter l'arrêt de la consommation de tabac, dans le traitement de la dépendance à la nicotine et/ou dans le traitement des symptômes du sevrage à la nicotine.

Selon la présente invention, on peut également utiliser le composé A, un de ses sels pharmaceutiquement acceptables ou un de leurs solvats, en association avec un autre principe actif, pour la préparation de médicaments utiles pour faciliter l'arrêt de la consommation de tabac, dans le traitement de la dépendance à la nicotine et/ou dans le traitement des symptômes du sevrage à la nicotine.

Par exemple le composé A peut être associé

- à la nicotine, un agoniste nicotinique ou un agoniste nicotinique partiel, ou bien
- à un inhibiteur de monoamine oxidase (IMAO),
- ou à tout autre principe actif ayant démontré une efficacité pour faciliter l'arrêt de la consommation de tabac, par exemple un antidépresseur tel que le bupropion, la doxepine, la nortriptyline ou un anxiolytique tel que la buspirone, ou bien la clonidine.

Pour son utilisation en tant que médicament, le composé A, un de ses sels pharmaceutiquement acceptables ou un de leurs solvats, seul ou en association avec un autre principe actif, doit être formulé en composition pharmaceutique.

Ainsi la présente invention a également pour objet des compositions pharmaceutiques contenant en association le N-pipéridino-5-(4-chlorophényl)-1-(2,4-dichlorophényl)-4-méthylpyrazole-3-carboxamide, un de ses sels pharmaceutiquement acceptables ou un de leurs solvats et un autre principe actif, l'autre principe actif étant un composé utile pour faciliter l'arrêt de la consommation de tabac, et/ou utile dans le traitement de la dépendance à la nicotine et/ou dans le traitement des symptômes du sevrage à la nicotine. Ledit autre principe actif étant préférentiellement choisi parmi :

- la nicotine, un agoniste nicotinique ou un agoniste nicotinique partiel, ou bien
- un inhibiteur de monoamine oxidase (IMAO),
- ou tout autre principe actif ayant démontré son efficacité pour faciliter l'arrêt de la consommation de tabac, par exemple un antidépresseur tel que le bupropion, la doxepine, la nortriptyline ou un anxiolytique tel que la buspirone, ou bien la clonidine.

Dans les compositions pharmaceutiques de la présente invention pour l'administration orale, sublinguale, sous-cutanée, intramusculaire, intraveineuse, transdermique, locale ou rectale, le principe actif, seul ou en association avec un autre principe actif, peut être administré sous forme unitaire d'administration, en mélange avec des supports pharmaceutiques classiques, aux animaux et aux êtres humains. Les formes unitaires d'administration appropriées comprennent les formes par voie orale telles que les comprimés, les gélules, les pilules, les poudres, les granules, les gommes à mâcher et les solutions ou suspensions orales, les formes d'administration sublinguale et buccale, les aérosols, les implants, les formes d'administration locale, transdermique, sous-cutanée, intramusculaire, intraveineuse, intranasale ou intraoculaire et les formes d'administration rectale.

Dans les compositions pharmaceutiques de la présente invention, le principe actif ou les principes actifs sont généralement formulés en unités de dosage. L'unité de dosage contient 0,5 à 300 mg, avantageusement de 5 à 60 mg, de préférence de 5 à 40 mg par unité de dosage, pour les administrations quotidiennes, une ou plusieurs fois par jour.

Bien que ces dosages soient des exemples de situations moyennes, il peut y avoir des cas particuliers où des dosages plus élevés ou plus faibles sont appropriés, de tels dosages appartiennent également à l'invention. Selon la pratique habituelle, le dosage approprié à chaque patient est déterminé par le médecin selon le mode d'administration, l'âge, le poids et la réponse dudit patient.

Lorsque l'on prépare une composition solide sous forme de comprimés, on peut ajouter au(x) principe(s) actif(s) micronisé(s) ou non un agent mouillant tel que le laurylsulfate de sodium et on mélange le tout avec un véhicule pharmaceutique tel que la silice, l'amidon, le lactose, le stéarate de magnésium, le talc ou analogues. On peut enrober les comprimés de saccharose, de divers polymères ou d'autres matières appropriées ou encore les traiter de telle sorte qu'ils aient une activité prolongée ou retardée et qu'ils libèrent d'une façon continue une quantité prédéterminée de principe actif.

On obtient une préparation en gélules en mélangeant le principe actif ou les principes actifs avec un diluant tel qu'un glycol ou un ester de glycérol et en incorporant le mélange obtenu dans des gélules molles ou dures.

Une préparation sous forme de sirop ou d'élixir peut contenir le principe actif ou les principes actifs conjointement avec un édulcorant, acalorique de préférence, du méthylparaben et du propylparaben comme antiseptiques, ainsi qu'un agent donnant du goût et un colorant approprié.

Les poudres ou les granules dispersibles dans l'eau peuvent contenir le principe actif ou les principes actifs en mélange avec des agents de dispersion ou des agents mouillants, ou des agents de mise en suspension, comme la polyvinylpyrrolidone ou polyvidone, de même qu'avec des édulcorants ou des correcteurs du goût.

Pour une administration rectale, on recourt à des suppositoires qui sont préparés avec des liants fondant à la température rectale, par exemple du beurre de cacao ou des polyéthylèneglycols.

Pour une administration parentérale, on utilise des suspensions aqueuses, des solutions salines isotoniques ou des solutions stériles et injectables qui contiennent des agents de dispersion et/ou des agents solubilisants pharmacologiquement compatibles, par exemple le propylèneglycol ou le butylèneglycol.

Ainsi, pour préparer une solution aqueuse injectable par voie intraveineuse on peut utiliser un cosolvant, par exemple un alcool tel que l'éthanol ou un glycol tel que le polyéthylèneglycol ou le propylèneglycol, et un tensioactif hydrophile tel que le polysorbate 80. Pour préparer une solution huileuse injectable par voie intramusculaire, on peut solubiliser le principe actif par un triglycéride ou un ester de glycérol.

Pour l'administration transdermique, on peut utiliser des patches sous forme multilaminée ou à réservoir dans lequel le principe actif est en solution alcoolique.

Le principe actif ou les principes actifs peuvent être formulés également sous forme de microcapsules ou microsphères, éventuellement avec un ou plusieurs supports ou additifs.

Le principe actif ou les principes actifs peuvent être également présentés sous forme de complexe avec une cyclodextrine, par exemple $\alpha$-, $\beta$- ou $\gamma$- cyclodextrine, 2-hydroxypropyl-$\beta$-cyclodextrine ou méthyl-$\beta$-cyclodextrine.

Parmi les formes à libération prolongée utiles dans le cas de traitements chroniques, on peut utiliser des implants. Ceux-ci peuvent être préparés sous forme de suspension huileuse ou sous forme de suspension de microsphères dans un milieu isotonique.

De façon préférentielle, le composé A est administré par la voie orale, en une prise unique par jour.

Selon un autre aspect de l'invention, le composé A, un de ses sels pharmaceutiquement acceptables ou un de leurs solvats et l'autre principe actif associé peuvent être administrés de manière simultanée, séparée ou étalée dans le temps pour faciliter l'arrêt de la consommation de tabac.

On entend par "utilisation simultanée" l'administration des composés de la composition selon l'invention compris dans une seule et même forme pharmaceutique.

On entend par "utilisation séparée" l'administration, en même temps, des deux composés de la composition selon l'invention chacun compris dans une forme pharmaceutique distincte.

On entend par "utilisation étalée dans le temps" l'administration successive, du premier composé de la composition selon l'invention, compris dans une forme pharmaceutique, puis, du deuxième composé de la composition selon l'invention, compris dans une forme pharmaceutique distincte.

Dans le cas de cette "utilisation étalée dans le temps", le laps de temps écoulé entre l'administration du premier composé de la composition selon l'invention et l'administration du deuxième composé de la même composition selon l'invention n'excède généralement pas 24 heures.

Les formes pharmaceutiques, comprenant soit un seul des composés constitutifs de la composition selon l'invention soit l'association des deux composés, qui peuvent être mises en oeuvre dans les différents types d'utilisations décrites ci-dessus, peuvent par exemple être appropriées à l'administration orale, nasale, parentérale ou transdermique.

Aussi, dans le cas d'une "utilisation séparée" et d'une "utilisation étalée dans le temps", deux formes pharmaceutiques distinctes peuvent être destinées à la même voie d'administration ou à une voie d'administration différente (orale et transdermique ou orale et nasale ou parentérale et transdermique).

L'invention concerne donc également une trousse pour faciliter l'arrêt de la consommation de tabac contenant le composé A et un autre principe actif facilitant l'arrêt de la consommation de tabac dans laquelle ledit composé A et ledit principe actif sont dans des compartiments distincts et dans des conditionnements semblables ou différents, et sont destinés à être administrés de manière simultanée, séparée ou étalée dans le temps. Ledit principe actif est préférentiellement choisi parmi :

- la nicotine, un agoniste nicotinique ou un agoniste nicotinique partiel, ou bien
- un inhibiteur de monoamine oxidase (IMAO),
- ou tout autre principe actif ayant démontré une efficacité pour faciliter l'arrêt de la consommation de tabac, par exemple un antidépresseur tel que le bupropion, la doxepine, la nortriptyline ou un anxiolytique tel que la buspirone, ou bien la clonidine.

Selon un autre de ses aspects, l'invention concerne aussi une méthode pour faciliter l'arrêt de la consommation de tabac qui consiste à administrer à un consommateur de nicotine une quantité thérapeutiquement efficace du composé A, d'un de ses sels pharmaceutiquement acceptables ou un de leurs solvats.

Les effets du composé A ont été étudiés chez le rat sur un modèle prédictif des effets sur la dépendance envers la nicotine : l'autoadministration de nicotine selon W.T. Corrigal and al. Psychopharmacology, 1989, 99, 473-478.

Le composé A, administré à la dose de 0,3 mg/kg et 1 mg/kg diminue de façon statistiquement significative le nombre d'injections de nicotine, chez des rats qui ont appris à s'autoadministrer de la nicotine par voie intraveineuse.

Ainsi, on a constaté les effets positifs du composé A sur ces 2 modèles.

Une étude en double aveugle a été réalisée avec des sujets fumant plus de 15 cigarettes par jour et montrant des symptômes de dépendance à la nicotine. Les patients reçoivent 40 mg du composé A par jour pendant 10 semaines dont 2 semaines avant le début de la période d'abstinence tabagique. On observe dans le groupe traité un taux d'abstinence plus important que dans le groupe recevant un placebo, notamment lors des 4 dernières semaines de traitement. L'abstinence tabagique est confirmée par la mesure hebdomadaire des taux de monoxide de carbone expiré et de cotinine plasmatique.

| EXEMPLE 1 : gélule dosée à 5 mg de composé A. | |
|---|---|
| Composé A micronisé | 5,00 mg |
| Amidon de maïs | 51,00 mg |
| Lactose monohydrate | 99,33 mg |

(suite)

| EXEMPLE 1 : gélule dosée à 5 mg de composé A. | |
|---|---|
| Polyvidone | 4,30 mg |
| Laurylsulfate de sodium | 0,17 mg |
| Carboxyméthyl cellulose de sodium réticulée | 8,50 mg |
| Eau purifiée : Q.S. pour granulation humide | |
| Stéarate de magnésium | 1,70 mg |
| Pour une gélule blanc opaque n° 3 remplie à | 170 mg |

| EXEMPLE 2 : gélule dosée à 10 mg de composé A. | |
|---|---|
| Composé A micronisé | 10,00 mg |
| Amidon de maïs | 51,00 mg |
| Lactose monohydrate | 94,33 mg |
| Polyvidone | 4,30 mg |
| Laurylsulfate de sodium | 0,17 mg |
| Carboxyméthyl cellulose de sodium réticulée | 8,50 mg |
| Eau purifiée : Q.S. pour granulation humide | |
| Stéarate de magnésium | 1,70 mg |
| Pour une gélule blanc opaque n° 3 remplie à | 170 mg |

| EXEMPLE 3 : gélule dosée à 20 mg de composé A. | |
|---|---|
| Composé A micronisé | 20,00 mg |
| Amidon de maïs | 51,00 mg |
| Lactose monohydrate | 84,33 mg |
| Polyvidone | 4,30 mg |
| Laurylsulfate de sodium | 0,17 mg |
| Carboxyméthyl cellulose de sodium réticulée | 8,50 mg |
| Eau purifiée : Q.S. pour granulation humide | |
| Stéarate de magnésium | 1,70 mg |
| Pour une gélule blanc opaque remplie à | 170 mg |

| EXEMPLE 4 : comprimé dosé à 10 mg de composé A. | |
|---|---|
| Composé A micronisé | 10,00 mg |
| Amidon de maïs | 50,00 mg |
| Lactose monohydrate 200 mesh | 211,50 mg |
| Hydroxypropylméthylcellulose 6 cP | 9,00 mg |
| Carboxyméthylamidon sodique | 15,00 mg |
| Laurylsulfate de sodium | 1,50 mg |
| Stéarate de magnésium | 3,00 mg |
| Eau purifiée : Q.S. | |
| Pour un comprimé terminé à | 300 mg |

| EXEMPLE 5 : comprimé dosé a 30 mg de composé A. | |
|---|---|
| Composé A micronisé | 30,00 mg |
| Amidon de maïs | 80,00 mg |
| Lactose monohydrate 200 mesh | 252,00 mg |
| Povidone K 30 | 12,00 mg |
| Carboxyméthylcellulose sodique réticulée | 20,00 mg |
| Laurylsulfate de sodium | 2,00 mg |
| Stéarate de magnésium | 4,00 mg |
| Eau purifiée : Q.S. | |
| Pour un comprimé terminé à | 400 mg |

## Revendications

1. Utilisation du N-pipéridino-5-(4-chlorophényl)-1-(2,4-dichlorophényl)-4-méthylpyrazole-3-carboxamide, d'un de ses sels pharmaceutiquement acceptables ou d'un de leurs solvats pour la préparation de médicaments destinés à faciliter l'arrêt de la consommation de tabac, au traitement de la dépendance à la nicotine et/ou au traitement des symptômes du sevrage à la nicotine.

2. Utilisation selon la revendication 1 du N-pipéridino-5-(4-chlorophényl)-1-(2,4-dichlorophényl)-4-méthylpyrazole-3-carboxamide, d'un de ses sels pharmaceutiquement acceptables ou d'un de leurs solvats en association avec un autre principe actif, dans laquelle l'autre principe actif est utile pour faciliter l'arrêt de la consommation de tabac et/ou utile dans le traitement de la dépendance nicotinique et/ou utile dans le traitement du sevrage à la nicotine.

3. Composition pharmaceutique contenant en association le N-pipéridino-5-(4-chlorophényl)-1-(2,4-dichlorophényl)-4-méthylpyrazole-3-carboxamide, un de ses sels pharmaceutiquement acceptables ou un de leurs solvats et un autre principe actif dans laquelle l'autre principe actif est utile pour faciliter l'arrêt de la consommation de tabac, et/ou utile dans le traitement de la dépendance à la nicotine et/ou dans le traitement des symptômes de sevrage à la nicotine.

4. Composition pharmaceutique selon la revendication 3 contenant le N-pipéridino-5-(4-chlorophényl)-1-(2,4-dichlorophényl)-4-méthylpyrazole-3-carboxamide, un de ses sels pharmaceutiquement acceptables ou un de leurs solvats et la nicotine, un agoniste nicotinique ou un agoniste nicotinique partiel.

5. Composition pharmaceutique selon la revendication 3 contenant le N-pipéridino-5-(4-chlorophényl)-1-(2,4-dichlorophényl)-4-méthylpyrazole-3-carboxamide, un de ses sels pharmaceutiquement acceptables ou un de leurs solvats et un inhibiteur de monoamine oxidase.

6. Trousse pour faciliter l'arrêt de la consommation de tabac contenant le N-pipéridino-5-(4-chlorophényl)-1-(2,4-dichlorophényl)-4-méthylpyrazole-3-carboxamide, un de ses sels pharmaceutiquement acceptables ou un de leurs solvats et un agoniste nicotinique ou un agoniste nicotinique partiel dans laquelle le N-pipéridino-5-(4-chlorophényl)-1-(2,4-dichlorophényl)-4-méthylpyrazole-3-carboxamide, un de ses sels pharmaceutiquement acceptables ou un de leurs solvats et ledit principe actif sont dans des compartiments distincts et dans des conditionnements semblables ou différents et sont destinés à être administrés de manière simultanée, séparée ou étalée dans le temps.

7. Trousse pour faciliter l'arrêt de la consommation de tabac contenant le N-pipéridino-5-(4-chtorophényl)-1-(2,4-dichlorophényl)-4-méthylpyrazole-3-carboxamide, un de ses sels pharmaceutiquement acceptables ou un de leurs solvats et un inhibiteur de la monoamine oxidase dans laquelle le N-pipéridino-5-(4-chlorophényl)-1-(2,4-dichlorophényl)-4-méthylpyrazole-3-carboxamide, un de ses sels pharmaceutiquement acceptables ou un de leurs solvats et ledit principe actif sont dans des compartiments distincts et dans des conditionnements semblables ou différents et sont destinés à être administrés de manière simultanée, séparée ou étalée dans le temps.

## Patentansprüche

1. Verwendung von N-Piperidino-5-(4-chlorphenyl)-1-(2,4-dichlorphenyl)-4-methylpyrazol-3-carboxamid, eines seiner pharmazeutisch annehmbaren Salze oder eines seiner Solvate für die Herstellung von Arzneimitteln, die dafür bestimmt sind, die Aufgabe des Tabakverbrauchs zu erleichtern, zur Behandlung der Nicotinabhängigkeit und/oder zur Behandlung von Symptomen des Nicotinentzugs.

2. Verwendung nach Anspruch 1 von N-Piperidino-5-(4-chlorphenyl)-1-(2,4-dichlorphenyl)-4-methylpyrazol-3-carboxamid, eines seiner pharmazeutisch annehmbaren Salze oder eines seiner Solvate in Kombination mit einem weiteren Wirkstoff, wobei der weitere Wirkstoff dazu geeignet ist, die Aufgabe des Nicotonverbrauchs zu erleichtern und/oder nützlich ist bei der Behandlung der Nicotinabhängigkeit und/oder bei der Behandlung des Nicotinentzugs.

3. Pharmazeutische Zubereitung enthaltend in Kombination N-Piperidino-5-(4-chlorphenyl)-1-(2,4-dichlorphenyl)-4-methylpyrazol-3-carboxamid, eines seiner pharmazeutisch annehmbaren Salze oder eines seiner Solvate und eines weiteren Wirkstoffs, wobei der weitere Wirkstoff nützlich ist für die Erleichterung der Aufgabe des Tabakverbrauchs und/oder nützlich ist bei der Behandlung der Nicotinabhängigkeit und/oder bei der Behandlung von Symptomen des Nicotinentzugs.

4. Pharmazeutische Zubereitung nach Anspruch 3, enthaltend N-Piperidino-5-(4-chlorphenyl)-1-(2,4-dichlorphenyl)-4-methylpyrazol-3-carboxamid, eines seiner pharmazeutisch annehmbaren Salze oder seiner Solvate und Nicotin, einen Nicotin-Agonisten oder einen Nicotin-Teilagonisten.

5. Pharmazeutische Zubereitung nach Anspruch 3, enthaltend N-Piperidino-5-(4-chlorphenyl)-1-(2,4-dichlorphenyl)-4-methylpyrazol-3-carboxamid, eines seiner pharmazeutisch annehmbaren Salze oder seiner Solvate und einen Inhibitor der Monoaminoxidase.

6. Kit zur Erleichterung der Aufgabe des Tabakverbrauchs, umfassend N-Piperidino-5-(4-chlorphenyl)-1-(2,4-dichlorphenyl)-4-methylpyrazol-3-carboxamid, eines seiner pharmazeutisch annehmbaren Salze oder eines seiner Solvate und einen Nicotin-Agonisten oder einen Nicotin-Teilagonisten, wobei N-Piperidino-5-(4-chlorphenyl)-1-(2,4-dichlorphenyl)-4-methylpyrazol-3-carboxamid, eines seiner pharmazeutisch annehmbaren Salze oder eines seiner Solvate und der genannte Wirkstoff in getrennten Behältern vorliegen und in gleichartiger oder unterschiedlicher Umgebung und die dafür bestimmt sind, gleichzeitig, getrennt oder zeitlich verschoben verabreicht zu werden.

7. Kit zur Erleichterung der Aufgabe des Tabakverbrauchs, umfassend N-Piperidino-5-(4-chlorphenyl)-1-(2,4-dichlorphenyl)-4-methylpyrazol-3-carboxamid, eines seiner pharmazeutisch annehmbaren Salze oder eines seiner Solvate und einen Inhibitor der Monoaminoxidase, wobei N-Piperidino-5-(4-chlorphenyl)-1-(2,4-dichlorphenyl)-4-methylpyrazol-3-carboxamid, eines seiner pharmazeutisch annehmbaren Salze oder eines seiner Solvate und der Wirkstoff in getrennten Behältern und unter gleichartigen oder verschiedenartigen Umgebungen vorliegen und dafür bestimmt sind, gleichzeitig, getrennt oder zeitlich verschoben verabreicht zu werden.

## Claims

1. Use of N-piperidino-5-(4-chlorophenyl)-1-(2,4-dichlorophenyl)-4-methylpyrazole-3-carboxamide, a pharmaceutically acceptable salt thereof or a solvate thereof for the preparation of medicinal products that are intended for helping to quit tobacco consumption, for the treatment of nicotine dependence and/or for the treatment of the symptoms of withdrawal from nicotine.

2. Use, according to Claim 1, of N-piperidino-5-(4-chlorophenyl)-1-(2,4-dichlorophenyl)-4-methylpyrazole-3-carboxamide, a pharmaceutically acceptable salt thereof or a solvate thereof, in combination with another active principle, in which the other active principle is useful for helping to quit tobacco comsumption and/or useful in the treatment of nicotine dependence and/or useful in the treatment of withdrawal from nicotine.

3. Pharmaceutical composition containing, in combination, N-piperidino-5-(4-chlorophenyl)-1-(2,4-dichlorophenyl)-4-methylpyrazole-3-carboxamide, a pharmaceutically acceptable salt thereof or a solvate thereof and another active principle in which the other active principle is useful for helping to quit tobacco consumption and/or useful in the treatment of nicotine dependence and/or in the treatment of the symptoms of withdrawal from nicotine.

4. Pharmaceutical composition according to Claim 3, containing N-piperidino-5-(4-chlorophenyl)-1-(2,4-dichlorophenyl)-4-methylpyrazole-3-carboxamide, a pharmaceutically acceptable salt thereof or a solvate thereof and nicotine, a nicotine agonist or a partial nicotine agonist.

5. Pharmaceutical composition according to Claim 3, containing N-piperidino-5-(4- chlorophenyl)-2-(2,4-dichlorophenyl)-4-methylpyrazole-3-carboxamide, a pharmaceutically acceptable salt thereof or a solvate thereof and a monoamine oxidase inhibitor.

6. Kit for helping to quit tobacco consumption, containing N-piperidino-5-(4-chlorophenyl)-1-(2,4-dichlorophenyl)-4-methylpyrazole-3-carboxamide, a pharmaceutically acceptable salt thereof or a solvate thereof and a nicotine agonist or a partial nicotine agonist, in which the N-piperidino-5-(4-chlorophenyl)-1-(2,4-dichlorophenyl)-4-methylpyrazole-3-carboxamide, a pharmaceutically acceptable salt thereof, a solvate thereof and the said active principle are in separate compartments and in packaging which may be identical or different and are intended to be administered simultaneously, separately or sequentially.

7. Kit for helping to quit tobacco consumption, containing N-piperidino-5-(4-chlorophenyl)-1-(2,4-dichlorophenyl)-4-methylpyrazole-3-carboxamide, a pharmaceutically acceptable salt thereof or a solvate thereof and a monoamine oxidase inhibitor, in which the N-piperidino-5-(4-chlorophenyl)-1-(2,4-dichlorophenyl)-4-methylpyrazole-3-carboxamide, a pharmaceutically acceptable salt thereof, a solvate thereof and the said active principle are in separate compartments and in packaging which may be identical or different and are intended to be administered simultaneously, separately or sequentially.